(19) Europäisches Patentamt
European Patent Office
Office européen des brevets

(11) Veröffentlichungsnummer: **0 028 318**
**B1**

# (12) EUROPÄISCHE PATENTSCHRIFT

(45) Veröffentlichungstag der Patentschrift: **22.12.82**

(51) Int. Cl.³: **C 08 G 65/22**, C 08 K 5/35, C 07 D 498/10, C 07 D 498/20

(21) Anmeldenummer: **80105991.6**

(22) Anmeldetag: **03.10.80**

(54) **Ether auf Basis von Polyalkyl-1-oxa-diazaspirodecanen, ihre Herstellung, ihre Verwendung, sowie mit ihnen stabilisierte synthetische Polymere.**

(30) Priorität: **10.10.79 DE 2941004**

(43) Veröffentlichungstag der Anmeldung: **13.05.81 Patentblatt 81/19**

(45) Bekanntmachung des Hinweises auf die Patenterteilung: **22.12.82 Patentblatt 82/51**

(84) Benannte Vertragsstaaten: **AT BE CH DE FR GB IT LI LU NL SE**

(56) Entgegenhaltungen: **keine**

(73) Patentinhaber: **HOECHST AKTIENGESELLSCHAFT, Postfach 80 03 20, D-6230 Frankfurt/Main 80 (DE)**

(72) Erfinder: **Wiezer, Hartmut, Dr., Hans-Fischer-Strasse 6, D-8906 Gersthofen (DE)**
Erfinder: **Pfahler, Gerhard, Dr., Karlsbader Strasse 27, D-8900 Augsburg (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Ether auf Basis von Polyalkyl-1-oxa-diazaspirodecanen, ihre Herstellung, ihre Verwendung, sowie mit ihnen stabilisierte synthetische Polymere

Die Erfindung betrifft neue Ether bzw. Polyether auf Polyalkyldiazaspirodecanbasis, ein Verfahren zu ihrer Herstellung und ihre Verwendung als hochwertige Lichtschutzmittel für die Stabilisierung von organischen Polymeren gegen Photooxidation.

Die neuen Verbindungen besitzen die allgemeine Formel (I)

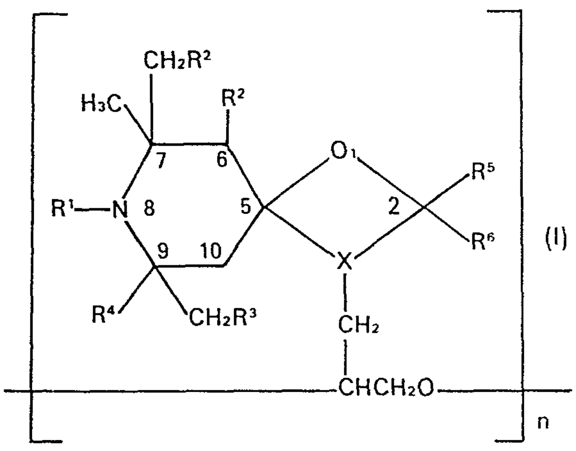

(I)

in welcher

n eine ganze Zahl von 1 bis 50, vorzugsweise von 1 bis 20 und insbesondere von 2 bis 15 ist, wobei im Falle von n = 1 über die freien Bindungen ein Oxiranring geschlossen ist,

X die Bedeutung einer Gruppe der Formel (II) oder (III) hat,

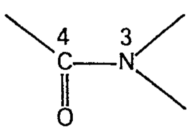

(II)

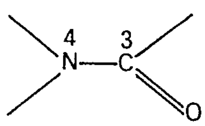

(III)

wobei die Indices 3 bzw. 4 die Ringpositionen im Diazaspirodecansystem angeben und eine Bindung des Stickstoffs mit einer $CH_2$-Gruppe des Etherrestes verknüpft ist,

$R^1$ Wasserstoff, Sauerstoff oder $C_1$- bis $C_{12}$-Alkyl, vorzugsweise Wasserstoff, Sauerstoff oder $C_1$- bis $C_4$-Alkyl und insbesondere Wasserstoff ist,

$R^2$ und $R^3$ entweder gleich sind und Wasserstoff oder eine $C_1$- bis $C_5$-Alkylgruppe, vorzugsweise Wasserstoff oder eine Methylgruppe und insbesondere Wasserstoff bedeuten, wobei dann

$R^4$ eine Methylgruppe ist, oder auch

$R^2$ Wasserstoff oder $C_1$- bis $C_5$-Alkyl ist und

$R^3$ und $R^4$ zusammen mit den Kohlenstoffatomen, an die sie gebunden sind, eine $C_5$- oder $C_6$-Cycloalkylgruppe oder eine Gruppe der Formel

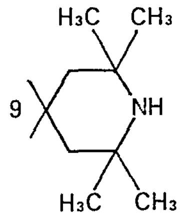

darstellen,

$R^5$ für Wasserstoff, $C_1$- bis $C_{30}$-Alkyl, vorzugsweise $C_1$- bis $C_{18}$-Alkyl und insbesondere $C_1$- bis $C_5$-Alkyl, für eine unsubstituierte oder durch Chlor oder $C_1$- bis $C_4$-Alkyl substituierte Phenyl- oder Naphthylgruppe, vorzugsweise die erstgenannte, oder für eine unsubstituierte oder durch $C_1$- bis $C_4$-Alkyl substituierte $C_7$- bis $C_{12}$-Phenylalkylgruppe, vorzugsweise für eine Benzylgruppe, steht,

$R^6$ Wasserstoff, $C_1$- bis $C_{30}$-Alkyl, vorzugsweise $C_1$- bis $C_{18}$-Alkyl und insbesondere $C_1$- bis $C_{13}$-Alkyl, eine unsubstituierte oder durch Chlor oder $C_1$- bis $C_4$-Alkyl substituierte Phenyl- oder Naphthylgruppe, vorzugsweise eine Phenylgruppe, eine unsubstituierte oder durch $C_1$- bis $C_4$-Alkyl-substituierte $C_7$- bis $C_{12}$-Phenylalkylgruppe, vorzugsweise eine Benzylgruppe ist, oder

$R^5$ und $R^6$ zusammen mit dem an sie gebundenen Kohlenstoffatom die Bedeutung einer unsubstituierten oder durch bis zu vier $C_1$- bis $C_4$-Alkylgruppen, vorzugsweise Methylgruppen substituierten $C_5$- bis $C_{18}$, vorzugsweise $C_5$- bis $C_{12}$-Cycloalkylgruppe oder einer Gruppe der Formel

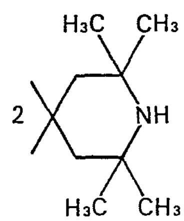

haben.

Beispiele für erfindungsgemässe Etherderivate der Formel (I) mit n = 1, aus welchen die Oligo- und Polyether gewonnen werden können, sind:

(1) 2,2,7,7,9,9-Hexamethyl-3-(2,3-epoxypropyl)-1-oxa-3,8-diaza-4-oxo-spiro-(4,5)-decan

(2) 2,2,7,7,9,9-Hexamethyl-4-(2,3-epoxypropyl)-1-oxa-3-oxo-4,8-diaza-spiro-(4,5)-decan

(3) 2,2,7,7,8,9,9-Heptamethyl-3-(2,3-epoxypropyl)-1-oxa-3,8-diaza-4-oxo-spiro-(4,5)-decan

(4) 2,2,7,7,8,9,9-Heptamethyl-4-(2,3-epoxypro-

pyl)-1-oxa-3-oxo-4,8-diaza-spiro-(4,5)-decan
(5) 2,7,7,9,9-Pentamethyl-2-ethyl-3-(2,3-epoxypropyl)-1-oxa-3,8-diaza-4-oxo-spiro-(4,5)-decan
(6) 2,7,7,9,9-Pentamethyl-2-propyl-3-(2,3-epoxypropyl)-1-oxa-3,8-diaza-4-oxo-spiro-(4,5)-decan
(7) 2,7,7,9,9-Pentamethyl-2-isopropyl-3-(2,3-epoxypropyl)-1-oxa-3,8-diaza-4-oxo-spiro-(4,5)-decan
(8) 2,7,7,9,9-Pentamethyl-2-butyl-3-(2,3-epoxypropyl)-1-oxa-3,8-diaza-4-oxo-spiro-(4,5)-decan
(9) 2,7,7,9,9-Pentamethyl-2-sek.-butyl-3-(2,3-epoxypropyl)-1-oxa-3,8-diaza-4-oxo-spiro-(4,5)-decan
(10) 2,7,7,9,9-Pentamethyl-2-pentyl-3-(2,3-epoxypropyl)-1-oxa-3,8-diaza-4-oxo-spiro-(4,5)-decan
(11) 2,7,7,9,9-Pentamethyl-2-hexyl-3-(2,3-epoxypropyl)-1-oxa-3,8-diaza-4-oxo-spiro-(4,5)-decan
(12) 2,7,7,9,9-Pentamethyl-2-(3-methylbutyl)-3-(2,3-epoxypropyl)-1-oxa-3,8-diaza-4-oxo-spiro-(4,5)-decan
(13) 7,7,9,9-Pentamethyl-2-ethyl-2-(2-methylbutyl)-3-(2,3-epoxypropyl)-1-oxa-3,8-diaza-4-oxo-spiro-(4,5)-decan
(14) 2,7,7,9,9-Pentamethyl-2-heptyl-3-(2,3-epoxypropyl)-1-oxa-3,8-diaza-4-oxo-spiro-(4,5)-decan
(15) 2,7,7,9,9-Pentamethyl-2-octyl-3-(2,3-epoxypropyl)-1-oxa-3,8-diaza-4-oxo-spiro-(4,5)-decan
(16) 2,7,7,9,9-Pentamethyl-2-nonyl-3-(2,3-epoxypropyl)-1-oxa-3,8-diaza-4-oxo-spiro-(4,5)-decan
(17) 2,7,7,9,9-Pentamethyl-2-undecyl-3-(2,3-epoxypropyl)-1-oxa-3,8-diaza-4-oxo-spiro-(4,5)-decan
(18) 2,7,7,9,9-Pentamethyl-2-octadecyl-3-(2,3-epoxypropyl)-1-oxa-3,8-diaza-4-oxo-spiro-(4,5)-decan
(19) 2,7,7,9,9-Pentamethyl-2-benzyl-3-(2,3-epoxypropyl)-1-oxa-3,8-diaza-4-oxo-spiro-(4,5)-decan
(20) 2,7,7,9,9-Pentamethyl-2-(2-phenylethyl)-3-(2,3-epoxypropyl)-1-oxa-3,8-diaza-4-oxo-spiro-(4,5)-decan
(21) 7,7,9,9-Tetramethyl-2,2-diethyl-3-(2,3-epoxypropyl)-1-oxa-3,8-diaza-4-oxo-spiro-(4,5)-decan
(22) 7,7,9,9-Tetramethyl-2-ethyl-2-propyl-3-(2,3-epoxypropyl)-1-oxa-3,8-diaza-4-oxo-spiro-(4,5)-decan
(23) 7,7,9,9-Tetramethyl-2-ethyl-2-butyl-3-(2,3-epoxypropyl)-1-oxa-3,8-diaza-4-oxo-spiro-(4,5)-decan
(24) 7,7,9,9-Tetramethyl-2-ethyl-2-pentyl-3-(2,3-epoxypropyl)-1-oxa-3,8-diaza-4-oxo-spiro-(4,5)-decan
(25) 7,7,9,9-Tetramethyl-2,8-dipropyl-3-(2,3-epoxypropyl)-1-oxa-3,8-diaza-4-oxo-spiro-(4,5)-decan
(26) 7,7,9,9-Tetramethyl-2,2-dibutyl-3-(2,3-epoxypropyl)-1-oxa-3,8-diaza-4-oxo-spiro-(4,5)-decan
(27) 7,7,9,9-Tetramethyl-2,2-dipentyl-3-(2,3-epoxypropyl)-1-oxa-3,8-diaza-4-oxo-spiro-(4,5)-decan
(28) 7,7,9,9-Tetramethyl-2,2-diheptyl-3-(2,3-epoxypropyl)-1-oxa-3,8-diaza-4-oxo-spiro-(4,5)-decan
(29) 7,7,9,9-Tetramethyl-2,2-dibenzyl-3-(2,3-epoxypropyl)-1-oxa-3,8-diaza-4-oxo-spiro-(4,5)-decan
(30) 2,7,7,9,9-Pentamethyl-2-(2-methyl-2-phenyl-propyl)-3-(2,3-epoxypropyl)-1-oxa-3,8-diaza-4-oxo-spiro-(4,5)-decan
(31) 7,7,9,9-Tetramethyl-2-methyl-3-(2,3-epoxypropyl)-1-oxa-3,8-diaza-4-oxo-spiro-(4,5)-decan
(32) 7,7,9,9-Tetramethyl-2-butyl-3-(2,3-epoxypropyl)-1-oxa-3,8-diaza-4-oxo-spiro-(4,5)-decan
(33) 7,7,9,9-Tetramethyl-2-iso-pentyl-3-(2,3-epoxypropyl)-1-oxa-3,8-diaza-4-oxo-spiro-(4,5)-decan
(34) 7,7,9,9-Tetramethyl-2-iso-heptyl-3-(2,3-epoxypropyl)-1-oxa-3,8-diaza-4-oxo-spiro-(4,5)-decan
(35) 7,7,9,9-Tetramethyl-2-iso-octyl-3-(2,3-epoxypropyl)-1-oxa-3,8-diaza-4-oxo-spiro-(4,5)-decan
(36) 7,7,9,9-Tetramethyl-2-iso-nonyl-3-(2,3-epoxypropyl)-1-oxa-3,8-diaza-4-oxo-spiro-(4,5)-decan
(37) 2,2,4,4-Tetramethyl-7-oxa-3,13-diaza-13-(2,3-epoxypropyl)-14-oxo-dispiro-(5,1,4,2)-tetradecan
(38) 2,2,4,4-Tetramethyl-7-oxa-3,13-diaza-10-tert.-butyl-13-(2,3-epoxypropyl)-14-oxo-dispiro-(5,1,4,2)-tetradecan
(39) 2,2,4,4-Tetramethyl-7-oxa-3,14-diaza-14-(2,3-epoxypropyl)-15-oxo-dispiro-(5,1,5,2)-pentadecan
(40) 2,2,4,4,10,12-Hexamethyl-7-oxa-3,14-diaza-14-(2,3-epoxypropyl)-15-oxo-dispiro-(5,1,5,2)-pentadecan
(41) 2,2,4,4,10,10,12-Heptamethyl-7-oxa-3,14-diaza-14-(2,3-epoxypropyl)-15-oxo-dispiro-(5,1,5,2)-pentadecan
(42) 2,2,4,4,10,10,12,12-Octamethyl-7-oxa-3,14-diaza-14-(2,3-epoxypropyl)-15-oxo-dispiro-(5,1,5,2)-pentadecan
(43) 2,2,4,4-Tetramethyl-7-oxa-3,20-diaza-20-(2,3-epoxypropyl)-21-oxo-dispiro-(5,1,11,2)-heneicosan
(44) 2,7,7,9,9-Pentamethyl-2-ethyl-1-oxa-3-oxo-4-(2,3-epoxypropyl)-4,8-diaza-spiro-(4,5)-decan
(45) 2,7,7,9,9-Pentamethyl-2-hexyl-1-oxa-3-oxo-4-(2,3-epoxypropyl)-4,8-diaza-spiro-(4,5)-decan
(46) 2,7,7,9,9-Pentamethyl-2-undecyl-1-oxa-3-oxo-4-(2,3-epoxypropyl)-4,8-diaza-spiro-(4,5)-decan
(47) 7,7,9,9-Tetramethyl-2,2-diethyl-1-oxa-3-oxo-4-(2,3-epoxypropyl)-4,8-diaza-spiro-(4,5)-decan

(48) 7,7,9,9-Tetramethyl-2,2-dibutyl-1-oxa-3-oxo-4-(2,3-epoxypropyl)-4,8-diaza-spiro-(4,5)-decan

(49) 7,7,9,9-Tetramethyl-2-ethyl-1-oxa-3-oxo-4-(2,3-epoxypropyl)-4,8-diaza-spiro-(4,5)-decan

(50) 7,7,9,9-Tetramethyl-2-pentyl-1-oxa-3-oxo-4-(2,3-epoxypropyl)-4,8-diaza-spiro-(4,5)-decan

(51) 7,7,9,9-Tetramethyl-2-iso-pentyl-1-oxa-3-oxo-4-(2,3-epoxypropyl)-4,8-diaza-spiro-(4,5)-decan

(52) 7,7,9,9-Tetramethyl-2-iso-heptyl-1-oxa-3-oxo-4-(2,3-epoxypropyl)-4,8-diaza-spiro-(4,5)-decan

(53) 2,2,4,4-Tetramethyl-7-oxa-3,14-diaza-14-(2,3-epoxypropyl)-13-oxo-dispiro-(5,1,4,2)-tetradecan

(54) 2,2,4,4-Tetramethyl-7-oxa-3,15-diaza-15-(2,3-epoxypropyl)-14-oxo-dispiro-(5,1,5,2)-pentadecan

(55) 2,2,4,4-Tetramethyl-7-oxa-3,12-diaza-21-(2,3-epoxypropyl)-20-oxo-dispiro-(5,1,5,2)-heneicosan

(56) 2,2,4,4,10,10,12,12-Octamethyl-7-oxa-3,11,14-triaza-14-(2,3-epoxypropyl)-15-oxo-dispiro-(5,1,5,2)-pentadecan

(57) 7,7,9,9-Tetramethyl-1-oxa-2-(2,4-dichlorphenyl)-3-(2,3-epoxypropyl)-3,8-diaza-4-oxo-spiro-(4,5)-decan

Die neuen Verbindungen mit n = 1 werden durch nukleophile Substitution des Halogenatoms in Epihalogenhydrin der Formel (V), wobei unter Halogen ein Chlor-, Brom- oder Jodatom, bevorzugt Chlor, zu verstehen ist, mit Polyalkyloxadiazaspirodecanen der Formel (IV) gemäss der folgenden Reaktionsgleichung unter Halogenwasserstoffabspaltung erhalten. Anschliessendes Erhitzen des Oxirans führt zur Bildung der Polyether mit $n > 1$.

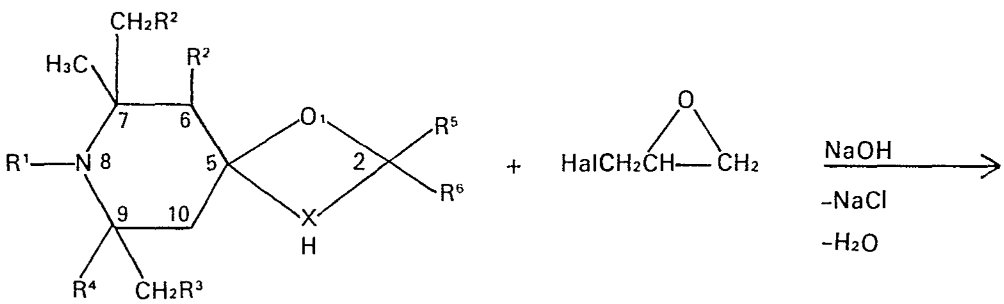


(IV) (V)

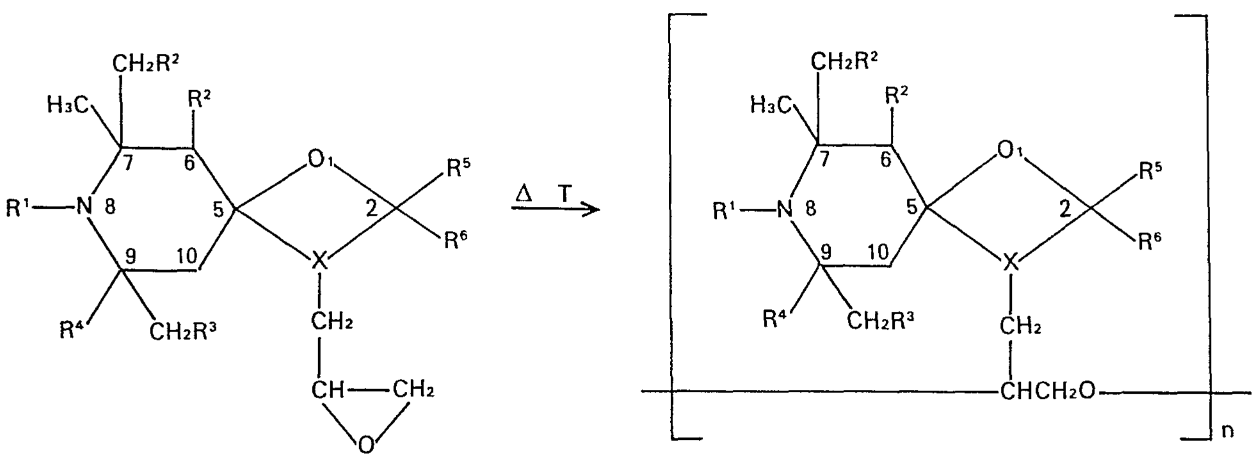


(I) mit n = 1 (I) mit $1 < n < 50$

In den Formeln des Reaktionsschemas haben die Reste $R^1$, $R^2$, $R^3$, $R^4$, $R^5$, $R^6$, X, Hal und n die oben angegebenen Bedeutungen.

Zur Synthese der Verbindungen mit n = 1 werden die Edukte (IV) und (V) im Molverhältnis 1:1 bis 1:5, vorzugsweise 1:1 bis 1:2 und insbesondere 1:1 bis 1:1,2 in einem inerten organischen Lösungsmittel bei Anwesenheit der äquimolaren bis zwanzigfach molaren Menge festen Alkalimetallhydroxids oder der entsprechenden Menge einer 20- bis 50%igen wässrigen Lösung eines solchen unter Einsatz eines Phasentransferkatalysators umgesetzt. Die Reaktionstemperatur liegt bei 20 bis 120, vorzugsweise 20 bis 80 und insbesondere 40 bis 60°C.

Als organische Lösungsmittel kommen aliphatische oder aromatische Kohlenwasserstoffe, wie z.B. Petrolether, Hexan, Heptan, Benzinschnitte, Toluol, Cyclohexan, Xylol etc. in Frage.

Unter Phasentransferkatalysatoren werden Substanzen aus der Gruppe der quartären Ammoniumhalogenide verstanden. Besonders gut ist Tricaprylmethylammoniumchlorid geeignet. Man benötigt 0,1 bis 5 Gew.-%, bezogen auf Verbindung (IV).

Die Reaktion ist im allgemeinen nach ein bis 20 Stunden beendet.

Zur Isolierung der Verbindungen mit n = 1 werden die Phasen, gegebenenfalls nach Zugabe von wenig Wasser, getrennt. Die organische Phase wird mehrfach mit Wasser gewaschen, über $Na_2SO_4$ oder $MgSO_4$ getrocknet und eingeengt. In den meisten Fällen resultieren ölige Produkte.

Aus den dergestalt erhaltenen Epoxiden lassen sich durch Erhitzen auf 100 bis 240, vorzugsweise 100 bis 200 und insbesondere 120 bis 180°C feste, amorphe, zunächst glasartig anfallende Polyether mit $1 < n < 50$ gewinnen. Niedrige Polymerisationsgrade können durch kurze und hohe durch lange Polymerisierdauer erzielt werden. Desgleichen wird mit steigender Temperatur die Tendenz zu höheren Polymerisationsgraden beobachtet.

Zur Herstellung der Polymeren bzw. Oligomeren kann man auch so vorgehen, dass man die Epoxide gar nicht erst isoliert, sondern die ganze Reaktionsmischung nach der Umsetzung des Epichlorhydrins mit dem Azaspirodecan auf die genannten höheren Temperaturen bringt und nach erfolgter Polymerisation aufarbeitet.

Da bei der Polymerisation stets Wasser vorhanden ist, dürften die Endgruppen der Polymerisate -H und -OH sein.

Die als Ausgangsprodukte verwendeten Polyalkyloxadiazaspirodecane sind bekannt und nach den in den DEA-2 606 026, 2 634 957 und 2 834 962 angegebenen Vorschriften zugänglich.

Der Ablauf der Reaktion in der beobachteten Weise ist überraschend und war nicht vorhersehbar. Insbesondere im Falle von Edukten (IV) mit $R^1$ = H war es keineswegs zu erwarten, dass der nukleophile Angriff auf das Epihalogenhydrin ausschliesslich am Amidstickstoff (Position 3 bzw. 4 des Ringsystems) erfolgt. Man musste vielmehr annehmen, dass der erheblich stärker nukleophile basische Aminostickstoff des Piperidinringes mit dem Epihalogenhydrin reagieren würde.

Die Möglichkeit, die primär erhaltenen Oxirane (Verbindungen I mit n = 1) zu Polyethern zu polymerisieren, konnte ebenfalls nicht vorhergesehen werden, da grundsätzlich auch eine Polyaddition durch Anlagerung des Aminstickstoffs (Position 8 des Ringsystems I) an die Epoxygruppe möglich erscheint.

Hochmolekulare Polyalkylpiperidinstabilisatoren sind bereits bekannt. Es handelt sich jedoch im Gegensatz zu den neuen, durch Polymerisation erhaltenen Polyethern um Additions- und Kondensationspolymere, welche Polyalkylpiperidinreste enthalten (DE-A-2 719 131, EP-A-1835, 2005 und 769). Von diesen polymeren Stabilisatoren gehört das Produkt EP-A-769, welches durch intermolekulare Polyaddition der Substanz nach Beispiel 2 der DE-A 2 233 122 – das ist das 3-(2,3-Epoxypropyl)-7,7,9,9-tetramethyl-1,3,8-triaza-spiro-(4,5)-decan-2,4-dion – erhalten wird, zum der vorliegenden Erfindung am nächsten kommenden Stand der Technik. Das zitierte Produkt nach Beispiel 2 der DE-A-2 233 122 ist unter gleichen Prüfbedingungen sehr viel schlechter wirksam als das Produkt nach Beispiel 58 der DE-A-2 227 689, das ebenfalls zum nächsten Stand der Technik zu zählen ist (Stabilität von Polypropylen mit Substanz nach Beispiel 2 750 Stdd., mit Substanz nach Beispiel 58 1420 Stdd.). Bei den anwendungstechnischen Prüfungen im Rahmen der vorliegenden Erfindung wurde daher als Vergleichssubstanz das Produkt nach DE-A-2 227 689 eingesetzt.

Die genannten bisher bekannten polymeren Stabilisatoren sind mit dem Mangel behaftet, dass sie nicht in allen wichtigen anwendungstechnischen Parametern, zu welchen neben der Wirksamkeit noch die Flüchtigkeit, Migrationsfestigkeit (gleichbedeutend mit geringer Auswaschbarkeit) und Thermostabilität zählen, den technischen Erfordernissen genügen. Demgegenüber erfüllen die neuen, erfindungsgemässen Stabilisatoren diese Anforderungen in hervorragender Weise. Sie besitzen eine sehr gute Stabilisatorwirksamkeit und sind, trotz der im Vergleich zu dem oben beschriebenen Stabilisator des Standes der Technik ähnlichen Struktur, weitgehend frei von auf physikalischen Eigenschaften beruhenden Nachteilen.

Unter Kunststoffen, die gegen eine Schädigung durch die Einwirkung von Sauerstoff, Wärme und Licht geschützt werden können, sind beispielsweise zu verstehen:

Polymere, die sich von einfach oder doppelt ungesättigten Kohlenwasserstoffen ableiten, z.B. Polyolefine wie Polyethylen, das gegebenenfalls vernetzt sein kann, Polypropylen, Polybuten-1, Polyisobuten, Polymethylbuten-1, Polymethylpenten-1, Polyisopren, Polybutadien, Polystyrol, Copolymere der den genannten Homopolymeren zugrundeliegenden Monomeren, wie Ethylen-Propylen-Copolymere, Propylen-Buten-1-Copolymere, Propylen-Isobuten-Copolymere, Styrol-Butadien-Copolymere, sowie Terpolymere von Ethylen und Propylen mit einem Dien, wie z.B. Hexadien, Dicyclopentadien oder Ethylidennorbornen; Mischungen der obengenannten Homopolymeren, wie beispielsweise Gemische von Polypropylen und Polyethylen, Polypropylen und Polybuten-1, Polypropylen und Polyisobutylen oder von Butadien-Acrylnitril-Copolymerisaten mit einem Styrol-Butadien-Copolymerisat.

Halogenhaltige Vinylpolymere wie Polyvinylchlorid, Polyvinylidenchlorid, Polyvinylfluorid, Polychloropren und Chlorkautschuke, sowie Copolymere von Vinylchlorid und Vinylidenchlorid untereinander und mit anderen olefinisch ungesättigten Monomeren.

Polymere, die sich von α,β-ungesättigten Säu-

ren und deren Derivaten ableiten, wie Polyacrylate und Polymethacrylate, Polyacrylamide und Polyacrylnitril sowie deren Copolymere untereinander und mit anderen Vinylverbindungen, wie Acrylnitril/Butadien/Styrol, Acrylnitril/Styrol und Acrylnitril/Styrol/Acrylester-Copolymerisate.

Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin und deren Copolymere mit anderen Vinylverbindungen, wie Ethylen/Vinylacetat-Copolymere.

Mono- und Copolymere, die sich von Epoxiden ableiten, wie Polyethylenoxid oder die Polymerisate, die sich von Bisglycidylethern ableiten.

Polyacetale, wie Polyoximethylen und Polyoxiethylen, sowie solche Polyoximethylene, die als Comonomere Ethylenoxid enthalten.

Polyurethane und Polyharnstoffe.

Polycarbonat.

Polyamide und Copolyamide, die sich von Diaminen und Dicarbonsäuren und/oder von Aminocarbonsäuren oder den entsprechenden Lactamen ableiten, wie Polyamid 6, Polyamid 6/6, Polyamid 6/10, Polyamid 11, Polyamid 12.

Polyester, die sich von Dicarbonsäuren und Dialkoholen und/oder von Hydroxicarbonsäuren oder den entsprechenden Lactonen ableiten, wie Polyethylenterephthalat, Polybutylenterephthalat, Poly-1,4-dimethylol-cyclohexanterephthalat.

Vernetzte Polymerisate, die sich von Aldehyden einerseits und Phenolen, Harnstoffen und Melaminen andererseits ableiten, wie Phenol-Formaldehyd-, Harnstoff-Formaldehyd- und Melamin-Formaldehydharze.

Von besonderer Bedeutung ist die Stabilisierung von Polyolefinen, Styrolpolymerisaten, Polyamiden, Poly(meth-)acrylaten und von Polyurethanen. Beispiele hierfür sind Polyethylen hoher und niedriger Dichte, Polypropylen, Ethylen-Propylen-Copolymerisate, Polystyrol, Styrol-Butadien-Acrylnitril-Tercopolymerisate, Mischungen von Polyolefinen oder von Styrolpolymerisaten, Polyurethane auf Polyether- oder Polyesterbasis in Form von Lacken, Fäden, Folien, Platten, Filmen, Elastomeren oder Schaumstoffen.

Die neuen Stabilisatoren werden nach allgemein üblichen Methoden in die Polymermassen eingearbeitet. Die Einarbeitung kann beispielsweise durch Einmischen der Verbindungen und gegebenenfalls weiterer Additive in die Schmelze vor oder während der Formgebung, oder auch durch Aufbringen der gelösten oder dispergierten Verbindungen auf das Polymere direkt oder Einmischen in eine Lösung, Suspension oder Emulsion desselben, gegebenenfalls unter nachträglichem Verdunstenlassen des Lösungsmittels erfolgen. Die den Kunststoffen zuzusetzenden Mengen liegen bei 0,05 bis 5, vorzugsweise 0,1 bis 2,5 und insbesondere 0,1 bis 1,0 Gew.-%, bezogen auf das zu stabilisierende Material.

Die neuen Verbindungen können auch in Form eines Masterbatches, der diese Verbindungen beispielsweise in einer Konzentration von 2,5 bis 50, vorzugsweise 5,0 bis 20 Gew.-% enthält, den zu stabilisierenden Kunststoffen zugesetzt werden.

Die zu stabilisierenden Kunststoffe können gegebenenfalls noch andere bekannte und übliche Zusätze enthalten. Genannt seien hier beispielsweise Antioxidantien auf Phenol- und Sulfidbasis, UV-Absorber und Lichtschutzmittel, Phosphitstabilisatoren, Metallverbindungen, Epoxistabilisatoren und mehrwertige Alkohole.

Beispiele für geeignete Antioxidantien sind sterisch gehinderte Phenole wie 4,4′-Butyliden-bis-(2,6-di-tert.-butyl-phenol), 4,4′-Thio-bis-(2-tert.-butyl-5-methylphenol), phenolische Triazinverbindungen, Thiodipropionsäureester von Fettalkoholen sowie Dioctadecylsulfit und -disulfid.

Zu den UV-Absorbern und Lichtschutzmitteln gehören z.B. 2-(2′-Hydroxyphenyl)-benztriazole wie 2-(2′-Hydroxy-5′-methylphenyl)-benztriazol, 2-Hydroxybenzophenone wie 2-Hydroxy-4-octoxybenzophenol, Stabilisatoren aus der Gruppe der Salizylate wie Octylphenylsalizylat, Nickelchelate, Oxalsäurediamide und sterisch gehinderte Piperidinverbindungen.

Als Phosphite sind Trisnonylphenylphosphit, Trislaurylphosphit oder auch Ester des Pentaerythritphosphits zu nennen.

Unter als Stabilisatoren bekannten Metallverbindungen werden in diesem Zusammenhang verstanden: Calcium-, Zink-, Barium-, Strontium-, Cadmium-, Magnesium-, Aluminium- und Bleiseifen aliphatischer Carbonsäuren oder Oxycarbonsäuren mit etwa 12 bis 32 C-Atomen, Salze der genannten Metalle mit aromatischen Carbonsäuren wie Benzoate oder Salizylate sowie (Alkyl)Phenolate dieser Metalle, ferner Organozinnverbindungen, wie z.B. Dialkylzinnthioglykolate und Carboxylate.

Bekannte Expoxistabilisatoren sind z.B. epoxidierte höhere Fettsäuren wie epoxidiertes Sojabohnenöl, Tallöl, Leinöl oder epoxidiertes Butyloleat sowie Epoxide langkettiger Olefine.

Mehrwertige Alkohole können beispielsweise Pentaerythrit, Trimethylolpropan, Sorbit oder Mannit sein, d.h. bevorzugt Alkohole mit 5 oder 6 C-Atomen und 2 bis 6 OH-Gruppen.

Eine wirksame Stabilisatorkombination für Poly-α-Olefine, wie z.B. Hoch-, Mittel- und Niederdruckpolymerisate von $C_2$- bis $C_4$-α-Olefinen, insbesondere Polyethylen und Polypropylen oder von Copolymerisaten derartiger α-Olefine besteht, bezogen auf 100 Gewichtsteile Polymere, beispielsweise aus 0,01 bis 5 Gewichtsteilen einer der erfindungsgemäss zu verwendenden Verbindungen, 0,05 bis 6 Gewichtsteilen eines phenolischen Stabilisators, gegebenenfalls 0,01 bis 5 Gewichtsteilen eines schwefelhaltigen Costabilisators sowie gegebenenfalls 0,01 bis 3 Gewichtsteilen einer basischen oder neutralen Metallseife, wie z.B. Calciumstearat oder Zinkstearat sowie gegebenenfalls 0,1 bis 5 Gewichtsteilen eines Phosphits und gegebenenfalls 0,01 bis 5 Gewichtsteilen eines bekannten UV-Stabilisators aus der Gruppe der Alkoxyhydroxybenzophenone, 4-Hydroxyphenylbenzotriazole, Benzylidenmalonsäuremononitrilester oder der sog. Quencher, wie z.B. Nickelchelate.

Die erfindungsgemäss stabilisierten Kunststoffe können in verschiedener Form angewendet werden, z.B. als Folien, Fasern, Bändchen, Profile oder als Bindemittel für Lacke, Klebstoffe oder Kitte.

Die nachfolgenden Beispiele dienen der weiteren Erläuterung des Erfindungsgegenstandes.

Beispiel 1

2,2,7,7,9,9-Hexamethyl-1-oxa-3-(2,3-epoxypropyl)-3,8-diaza-4-oxo-spiro-(4,5)-decan und daraus erhaltene Oligomer

Zu 150 ml Toluol wurden nacheinander 24,0 g (0,1 Mol) 2,2,7,7,9,9-Hexamethyl-1-oxa-3,8-diaza-4-oxo-spiro-(4,5)-decan, 18,5 g (0,2 Mol) Epichlorhydrin, 5 Tropfen Tricaprylmethylammoniumchlorid (®Aliquat 336 der Fa. Fluka) und 40 g 50%ige Natronlauge (≙ 0,5 Mol NaOH) gegeben, worauf man das Reaktionsgemisch 16 Stunden bei 65°C rührte. Nach dem Abstellen des Rührers bildeten sich zwei klare Phasen, welche getrennt wurden. Die organische Phase wurde dreimal mit 50 ml Wasser gewaschen, über 50 g Natriumsulfat getrocknet, 30 Min. mit 1 g Aktivkohle bei Raumtemperatur verrührt und filtriert. Im Vakuum wurden die flüchtigen Anteile abgezogen. Es blieb ein farbloses Öl zurück, welches die in der Überschrift angegebene Epoxiverbindung ist. Sie wurde drei Stunden auf 170°C erwärmt und polymerisierte dabei zu einem festen, farblosen Harz vom Fp. 130 bis 184°C. Die relative spezifische Viskosität (RSV-Wert) bei 25°C in einer 1 Gew.-%igen Lösung in Toluol betrug 0,03.

Beispiele 2 bis 44

Es wurde wie in Beispiel 1 angegeben gearbeitet. In der nachstehenden Tabelle sind die Versuchsbedingungen sowie Angaben über die monomeren und polymeren Verfahrensprodukte zusammengestellt. Spalte 2 («Verbindung Nr.») verweist auf die Aufstellung typischer monomerer Verfahrensprodukte in der Beschreibung S. 3 bis 6, woraus sich auch das jeweils eingesetzte Polyalkyldiazaspirodecan ergibt.

| Bsp. Nr. | Verb. Nr. | Herstellung der Epoxiverbindung Zeit (h) | Temp. (°C) | Fp. der Epoxiverbindung (°C) | Polymerisation Zeit (h) | Temp. (°C) | Polymerisat Fp. (°C) | RSV-Wert |
|---|---|---|---|---|---|---|---|---|
| 2 | 2 | 15 | 60 | 175 | 6 | 170 | 142–183 | 0,02 |
| 3 | 4 | 10 | 60 | Öl | 6 | 150 | 56–88 | 0,05 |
| 4 | 5 | 16 | 50 | Öl | 6 | 170 | 59–92 | 0,03 |
| 5 | 6 | 9 | 60 | Öl | 3 | 175 | 102–154 | 0,02 |
| 6 | 7 | 19 | 65 | Öl | 3 | 170 | 99–171 | 0,02 |
| 7 | 7 | 19 | 65 | Öl | 6 | 170 | 140–196 | 0,02 |
| 8 | 9 | 15 | 70 | Öl | 3 | 170 | 100–149 | 0,02 |
| 9 | 9 | 15 | 70 | Öl | 6 | 170 | 134–188 | 0,02 |
| 10 | 10 | 20 | 57 | Öl | 6 | 150 | 84–138 | 0,03 |
| 11 | 11 | 13 | 60 | Öl | 1 | 200 | 95–121 | 0,02 |
| 12 | 12 | 7 | 66 | Öl | 5 | 180 | 98–161 | 0,03 |
| 13 | 13 | 11 | 60 | Öl | 5 | 170 | 91–141 | 0,04 |
| 14 | 14 | 15 | 60 | Öl | 1 | 200 | 55–128 | 0,02 |
| 15 | 45 | 15 | 60 | Öl | 5 | 170 | 80–119 | 0,03 |
| 16 | 54 | 13 | 55 | 95–99 | 5 | 170 | 168–229 | 0,02 |
| 17 | 1 | 16 | 65 | Öl | 6 | 170 | 151–208 | 0,04 |
| 18 | 56 | 18 | 50 | 165 | 6 | 170 | 55–191 | 0,03 |
| 19 | 19 | 15 | 60 | Öl | 6 | 180 | 128–149 | 0,04 |
| 20 | 20 | 15 | 50 | Öl | 6 | 150 | 74–117 | 0,02 |
| 21 | 21 | 16 | 55 | Öl | 3 | 170 | 86–139 | 0,02 |
| 22 | 21 | 16 | 55 | Öl | 6 | 170 | 148–201 | 0,02 |
| 23 | 23 | 14 | 60 | Öl | 6 | 150 | 105–137 | 0,02 |
| 24 | 24 | 3 | 60 | Öl | 3 | 180 | 84–134 | 0,03 |
| 25 | 25 | 16 | 65 | Öl | 6 | 150 | 66–103 | 0,02 |
| 26 | 26 | 20 | 60 | Öl | 5 | 170 | 60–116 | 0,03 |
| 27 | 27 | 8 | 50 | Öl | 3 | 150 | 72–124 | 0,03 |
| 28 | 28 | 14 | 40 | Öl | 3 | 150 | 57–94 | 0,03 |
| 29 | 29 | 14 | 60 | Öl | 6 | 180 | 144–170 | 0,03 |
| 30 | 42 | 13 | 50 | Öl | 6 | 170 | 72–96 | 0,03 |
| 31 | 30 | 16 | 60 | Öl | 1 | 200 | 123–176 | 0,02 |
| 32 | 37 | 14 | 70 | Öl | 1,5 | 190 | 138–189 | 0,02 |
| 33 | 37 | 14 | 70 | Öl | 3 | 170 | 131–174 | 0,02 |
| 34 | 38 | 8 | 55 | Öl | 3 | 170 | 136–198 | 0,02 |
| 35 | 39 | 15 | 60 | Öl | 3 | 170 | 138–190 | 0,01 |
| 36 | 39 | 15 | 60 | Öl | 6 | 170 | 172–247 | 0,03 |
| 37 | 40 | 13 | 50 | Öl | 6 | 170 | 65–84 | 0,02 |

| Bsp. Nr. | Verb. Nr. | Herstellung der Epoxiverbindung Zeit (h) | Temp. (°C) | Fp. der Epoxiverbindung (°C) | Polymerisation Zeit (h) | Temp. (°C) | Polymerisat Fp. (°C) | RSV-Wert |
|---|---|---|---|---|---|---|---|---|
| 38 | 41 | 14 | 50 | Öl | 1 | 200 | 129–166 | 0,01 |
| 39 | 42 | 13 | 50 | Öl | 6 | 170 | 72–96 | 0,03 |
| 40 | 43 | 6 | 50 | 138 | 3 | 170 | 101–163 | 0,02 |
| 41 | 43 | 12 | 60 | Öl | 6 | 170 | 129–177 | 0,03 |
| 42 | 50 | 14 | 55 | Öl | 6 | 170 | 57–78 | 0,02 |
| 43 | 51 | 9 | 60 | Öl | 6 | 170 | 78–111 | 0,03 |
| 44 | 57 | 20 | 50 | Öl | 6 | 170 | 58–86 | 0,04 |

Beispiel 45

Dieses Beispiel zeigt die Flüchtigkeit der neuen Stabilisatoren im Vergleich zu Produkten der DE-A-2 227 689.

Die Flüchtigkeiten wurden in einer Apparatur zur thermogravimetrischen Analyse bestimmt. Gleiche Mengen (500 mg) der erfindungsgemässen und der Verlgeichssubstanzen wurden dazu in Stickstoffatmosphäre mit einer Geschwindigkeit von 2 K/min bis auf 300°C aufgeheizt und der Substanzverlust in mg/cm² gemessen. Die Ergebnisse zeigt nachfolgende Tabelle:

| Stabilisator gemäss Beisp. | Gewichtsverlust in mg/cm² beim Erreichen von . . . °C 220 | 260 | 300 | 10 Min. bei 300 |
|---|---|---|---|---|
| 1 | 0,32 | 0,63 | 3,16 | 9,80 |
| 2 | 0,16 | 2,21 | 6,64 | 16,91 |
| 6 | 0,16 | 0,47 | 1,42 | 3,63 |
| 7 | 0,32 | 1,26 | 4,27 | 9,48 |
| 8 | 0,16 | 0,47 | 2,05 | 7,74 |
| 9 | 0,16 | 0,63 | 2,53 | 9,01 |
| 17 | 0,16 | 0,47 | 2,21 | 8,37 |
| 21 | 0,32 | 0,79 | 2,21 | 5,37 |
| 22 | 0,00 | 0,16 | 1,74 | 4,58 |
| 32 | 0,00 | 0,47 | 4,27 | 11,69 |
| 33 | 0,00 | 0,36 | 6,64 | 17,22 |
| 35 | 0,00 | 0,16 | 0,95 | 3,48 |
| 36 | 0,00 | 0,32 | 1,26 | 3,79 |
| 40 | 0,32 | 1,26 | 2,84 | 7,90 |
| 40 (Monomeres) | 0,48 | 2,25 | 3,00 | 7,11 |
| 41 | 0,16 | 0,47 | 1,90 | 6,64 |
| Vergleich+) | 14,06 | 45,82 | 148,52 | 153,26 |

+) Bsp. 58 der De-A-2 227 689

Beispiel 46

Zum Nachweis der stabilisierenden Eigenschaften der neuen Verbindungen wurde wie folgt verfahren:

100 Gew.-Teile Polypropylen mit einem Schmelzindex $i_5$ von ca. 6 g/10 min (bestimmt nach ASTM D 1238-62 T) und einer Dichte von 0,90 wurden mit

0,1 Gew.-Teilen Pentaerythrityl-tetrakis-3-(3,5-di-tert.-butyl-4-hydroxyphenyl)-propionat,

0,2 Gew.-Teilen Calciumstearat und

0,3 Gew.-Teilen des zu prüfenden erfindungsgemässen Stabilisators

vermischt.

Um eine möglichst gleichmässige Verteilung auf dem Polymerkorn zu erreichen, wurden die Stabilisatoren in einem Lösemittel gelöst. Die Lösung wurde unter Rühren in das Polypropylenpulver eingetropft, wobei durch gleichzeitige Bestrahlung mit einer IR-Lampe der grösste Teil des Lösemittels wieder abdampfte. Nach ca. 20 min wurde das Calciumstearat hinzugegeben und noch weitere 10 min gemischt. Lösemittelreste wurden durch Trocknen bei 50°C/120 min im Trockenschrank entfernt.

Das Polypropylen wurde auf einer Windsor-Spritzgussmaschine der Type SP 50 bei 240°C zu 60×60×1 mm-Platten verspritzt. Aus diesen Platten wurden Prüfkörper nach DIN 53455, Form 3, verkleinert im Massstab 1:3, ausgestanzt. Die als Vergleichsmuster benötigten Prüfkörper wurden analog, jedoch unter Fortlassen des zu testenden Stabilisators bzw. unter Zusatz der Vergleichsstabilisatoren, hergestellt.

Zur Bestimmung der Lichtstabilität wurden die Proben in einer Xenotest-120-Apparatur der Firma Original Hanau Quarzlampen GmbH der Bestrahlung mit Wechsellicht unterworfen. Die Strahlungsintensität wurde durch UV-Filter (Spezialfilterglas d = 1,7 mm) moduliert. Die Lichtbeständigkeit wurde nach DIN 53387 (17 min befeuchten, 3 min beregnen, Schwarztafeltemperatur 45°C, Luftfeuchtigkeit 70 bis 75%) geprüft. Gemessen wurde die Belichtungszeit in Stunden und die Reissdehnung bestimmt. Die Reissdehnung wurde auf einer Zugprüfmaschine der Firma Instron bei einer Abzugsgeschwindigkeit von 5 cm/min ermittelt.

| Stabilisator nach Beisp. | Belichtungszeit in Stunden | Ermittelte Reissdehnung in % vom Ausgangswert |
|---|---|---|
| 2 | 1100 | > 50 |
| 8 | 1100 | > 50 |
| 22 | 1100 | > 50 |
| 35 | 1100 | > 50 |
| 41 | 1100 | > 50 |
| 54 | 1100 | > 50 |
| Polypropylen | 260 | 1 |
| Vergleich ohne Stab. | 320 | 1 |
| Vergleich+) | 1100 | 2 |

+) Verbindung nach Beispiel 58 der DE-A-2 227 689

Beispiel 47

Zu Polypropylen (Hostalen PPU VP 1770 F der Hoechst AG) vom Schmelzindex MFI 190/51,9/10 min s. DIN 53535 werden 0,26 Gew.-Teile der unten angegebenen Stabilisatoren über einen Laborschnellmischer eingemischt. Das so stabilisierte Material wurde in einem Laborextruder unter den üblichen Verarbeitungsbedingungen aufgeschmolzen und über eine Spinnpumpe mit Mehrfachspinnkopf zu Monifilamenten (87 dtex) verarbeitet, welche anschliessend im Verhältnis 1:2,5 nachverstreckt wurden. Je 24 dieser Filamente wurden zu Garn texturiert und dieses zu Prüfgeweben verarbeitet. Die Prüflinge wurden in einem Fadeometer der Lichtechtheitsprüfung unterzogen und nach der angegebenen Belichtungszeit dem Fingernageltest (leichtes Darüberreiben über das Gewebe mit dem Daumennagel) unterzogen. Der Grad des Abbaus wird durch Wertzahlen ausgedrückt, wobei 0 = keine Schädigung und 1 bis 5 = zunehmende Zerstörbarkeit bedeuten.

| Stabilisator gemäss Beisp. | Zerstörbarkeit des Gewebes nach ... Std. Belichtungszeit 40 | 80 | 160 |
|---|---|---|---|
| ohne Stabilisator (Vergleich) | 0 | 0 | 5 |
| 21 | 0 | 0 | 0 |
| 41 | 0 | 0 | 0 |
| Vergleich+) | 0 | 0 | 1 |

+) Stabilisator gemäss Beispiel 58 der DE-A-2 227 689

**Patentansprüche** für die Vertragsstaaten: BE, CH, LI, DE, FR, GB, IT, LU, NL, SE

1. Polyalkylpiperidingruppenhaltige Ether der Formel (I)

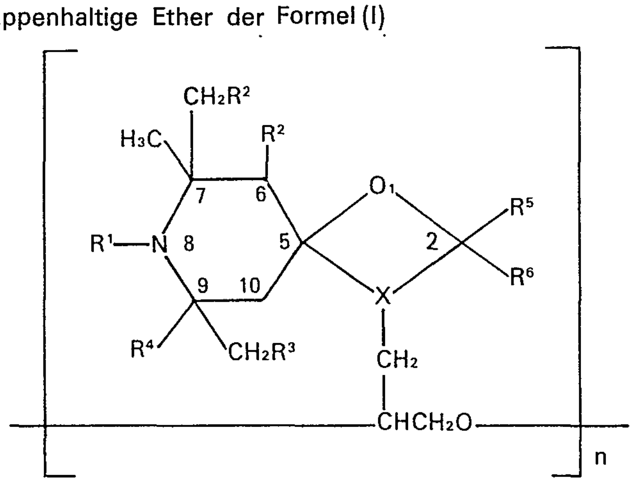

(I)

in welcher

n eine ganze Zahl von 1 bis 50 bedeutet, wobei im Falle von n = 1 über die freien Bindungen ein Oxiranring geschlossen ist,

X die Bedeutung einer Gruppe der Formel (II) oder (III) hat,

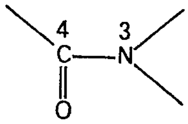 (II) 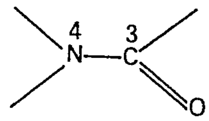 (III)

wobei die Indices 3 bzw. 4 die Ringpositionen im Diazaspirodecansystem angegeben und eine Bindung des Stickstoffs mit der $CH_2$-Gruppe des Etherrestes verknüpft ist,

$R^1$ Wasserstoff, Sauerstoff oder $C_1$- bis $C_{12}$-Alkyl ist,

$R^2$ und $R^3$ entweder gleich sind und Wasserstoff oder eine $C_1$- bis $C_5$-Alkylgruppe bedeuten, wobei dann

$R^4$ eine Methylgruppe ist, oder auch

$R^2$ Wasserstoff oder $C_1$- bis $C_5$-Alkyl ist und

$R^3$ und $R^4$ zusammen mit den Kohlenstoffatomen, an die sie gebunden sind, eine $C_5$- oder $C_6$-Cycloalkylgruppe oder eine Gruppe der Formel

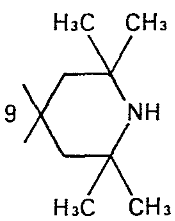

darstellen,

$R^5$ und $R^6$ gleich oder verschieden sind und für Wasserstoff, $C_1$- bis $C_{30}$-Alkyl, für eine unsubstituierte oder durch Chlor oder $C_1$- bis $C_4$-Alkyl substituierte Phenyl- oder Naphthylgruppe oder für eine unsubstituierte oder durch $C_1$- bis $C_4$-alkylsubstituierte $C_7$- bis $C_{12}$-Phenylalkylgruppe stehen, oder

$R^5$ und $R^6$ zusammen mit dem an sie gebundenen Kohlenstoffatom die Bedeutung einer unsubstituierten oder durch bis zu vier $C_1$- bis $C_4$-Alkylgruppen substituierten $C_5$- bis $C_{18}$-Cycloalkylgruppe oder einer Gruppe der Formel

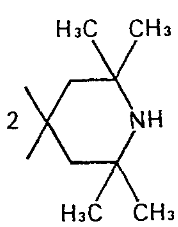

haben.

2. Verfahren zur Herstellung der Verbindungen nach Anspruch 1, dadurch gekennzeichnet, dass ein Polyalkyl-1-oxa-diaza-spirodecan der Formel (IV)

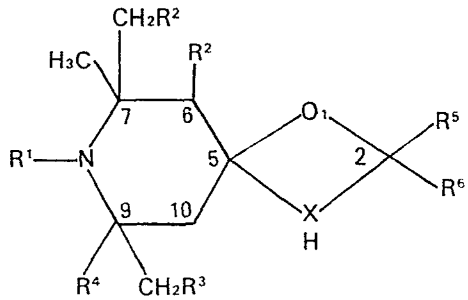 (IV)

in der $R^1$, $R^2$, $R^3$, $R^4$, $R^5$, $R^6$ und X die in Anspruch 1 angegebenen Bedeutungen haben, mit einem Epihalogenhydrin im Molverhältnis 1:1 bis 1:5 in einem Zweiphasensystem, bestehend aus einem organischen Lösungsmittel und einem Alkalimetallhydroxid in fester Form oder wässriger Lösung, in Anwesenheit von 0,1 bis 5 Gew.-%, bezogen auf Verbindung (IV), eines quartären Ammoniumhalogenids bei 20 bis 120°C umgesetzt wird, wobei Verbindungen mit n = 1 in Form des Oxirans resultieren, worauf man diese, falls gewünscht, bei 100 bis 240°C zu den Ethern mit n = 2 bis 50 polymerisiert.

3. Verwendung der Verbindungen nach Anspruch 1 zum Stabilisieren von synthetischen Polymeren gegen den schädigenden Einfluss von Licht, gegebenenfalls neben bisher bekannten, stabilisierend wirkenden Stoffen, in Mengen von 0,01 bis 5 Gewichtsteilen, bezogen auf das Polymere.

4. Verwendung nach Anspruch 3, dadurch gekennzeichnet, dass das Polymer ein Polyolefin, ein halogenhaltiges Polymer, ein Polyacrylat oder Polymethacrylat oder ein Homo- oder Copolymer des Styrols ist.

5. Gegen UV-Zersetzung stabilisierte synthetische Polymere, in denen 0,01 bis 5 Gewichtsteile, bezogen auf Polymere, einer Verbindung nach Anspruch 1 enthalten sind.

**Patentansprüche** für den Vertragsstaat: AT

1. Verfahren zur Herstellung von Polyalkylpiperidingruppenhaltigen Ethern der Formel (I)

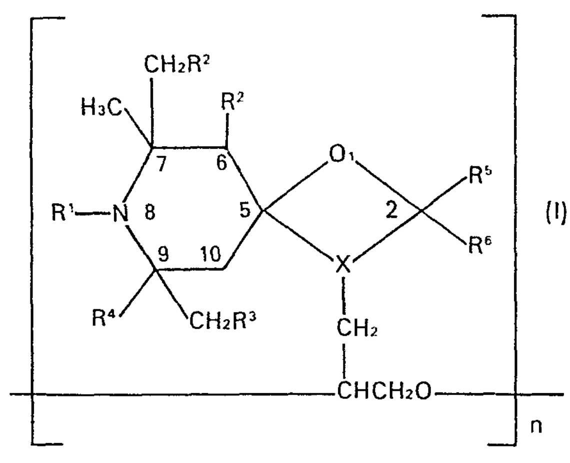 (I)

in welcher
n eine ganze Zahl von 1 bis 50 bedeutet, wobei im Falle von n = 1 über die freien Bindungen ein Oxiranring geschlossen ist,

X die Bedeutung einer Gruppe der Formel (II) oder (III) hat,

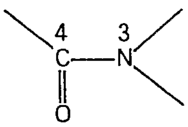
(II)
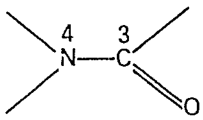
(III)

wobei die Indices 3 bzw. 4 die Ringpositionen im Diazaspirodecansystem angegeben und eine Bindung des Stickstoffs mit der $CH_2$-Gruppe des Etherrestes verknüpft ist,

$R^1$ Wasserstoff, Sauerstoff oder $C_1$- bis $C_{12}$-Alkyl ist,

$R^2$ und $R^3$ entweder gleich sind und Wasserstoff oder eine $C_1$- bis $C_5$-Alkylgruppe bedeuten, wobei dann

$R^4$ eine Methylgruppe ist, oder auch

$R^2$ Wasserstoff oder $C_1$- bis $C_5$-Alkyl ist und

$R^3$ und $R^4$ zusammen mit den Kohlenstoffatomen, an die sie gebunden sind, eine $C_5$- oder $C_6$-Cycloalkylgruppe oder eine Gruppe der Formel

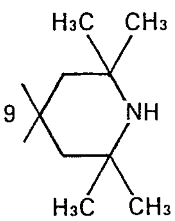

darstellen,

$R^5$ und $R^6$ gleich oder verschieden sind und für Waserstoff, $C_1$- bis $C^3{}_o$-Alkyl, für eine unsubstituierte oder durch Chlor oder $C_1$- bis $C_4$-Alkyl substituierte Phenyl- oder Naphthylgruppe oder für eine unsubstituierte oder durch $C_1$- bis $C_4$-alkylsubstituierte $C_7$- bis $C_{12}$-Phenylalkylgruppe stehen, oder

$R^5$ und $R^6$ zusammen mit dem an sie gebundenen Kohlenstoffatom die Bedeutung einer unsubstituierten oder durch bis zu vier $C_1$- bis $C_4$-Alkylgruppen substituierten $C_5$- bis $C_{18}$-Cycloalkylgruppe oder einer Gruppe der Formel

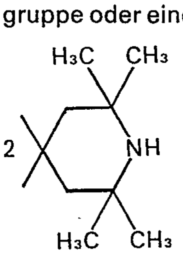

haben,

dadurch gekennzeichnet, dass ein Polyalkyl-1-oxa-diaza-spirodecan der Formel (IV)

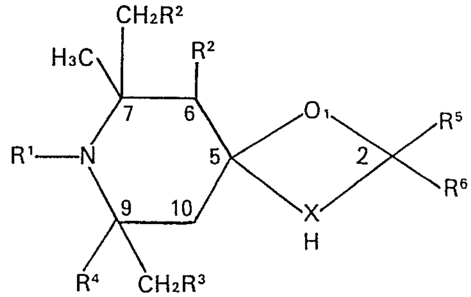
(IV)

in der $R^1$, $R^2$, $R^3$, $R^4$, $R^5$, $R^6$ und X die oben angegebenen Bedeutungen haben, mit einem Epihalogenhydrin im Molverhältnis 1:1 bis 1:5 in einem Zweiphasensystem, bestehend aus einem organischen Lösungsmittel und einem Alkalimetallhydroxid in fester Form oder wässriger Lösung, in Anwesenheit von 0,1 bis 5 Gew.-%, bezogen auf Verbindung (IV), eines quartären Ammoniumhalogenids bei 20 bis 120°C umgesetzt wird, wobei Verbindungen mit n = 1 in Form des Oxirans resultieren, worauf man diese, falls gewünscht, bei 100 bis 240°C zu den Ethern mit n = 2 bis 50 polymerisiert.

2. Verwendung der nach Anspruch 1 hergestellten Verbindungen zum Stabilisieren von synthetischen Polymeren gegen den schädigenden Einfluss von Licht, gegebenenfalls neben bisher bekannten, stabilisierend wirkenden Stoffen, in Mengen von 0,01 bis 5 Gewichtsteilen, bezogen auf das Polymer.

3. Verwendung nach Anspruch 2, dadurch gekennzeichnet, dass das Polymer ein Polyolefin, ein halogenhaltiges Polymer, ein Polyacrylat oder Polymethacrylat oder ein Homo- oder Copolymer des Styrols ist.

4. Gegen UV-Zersetzung stabilisierte synthetische Polymere, in denen 0,01 bis 5 Gewichtsteile, bezogen auf Polymer, einer Verbindung nach Anspruch 1 enthalten sind.

**Claims** for the Contracting states: BE, CH, DE, FR, GB, IT, LI, LU, NL, SE

1. Ethers, containing polyalkylpiperidine groups, of the formula (I)

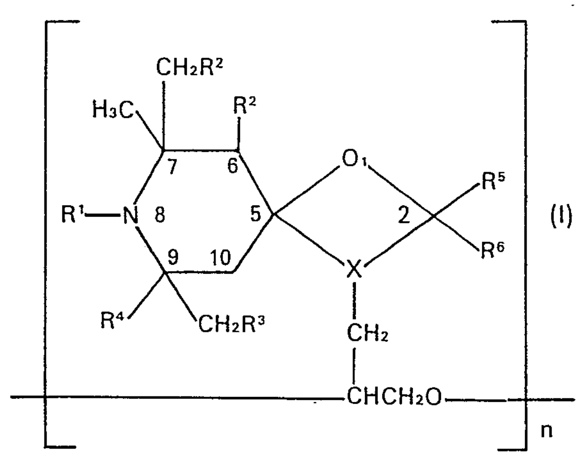
(I)

in which n is an integer from 1 to 50, when n equals 1, an oxirane ring being completed via the free bonds, X denotes a group of the formula (II) or (III)

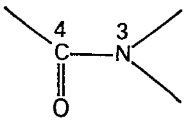 (II) 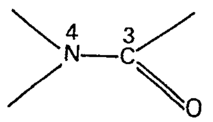 (III)

wherein the indices 3 and 4 indicate the ring positions in the diazaspirodecane system and one bond of the nitrogen is attached to the $CH_2$ group of the ether radial, $R^1$ is hydrogen, oxygen or $C_1$- to $C_{12}$-alkyl, $R^2$ and $R^3$ are either identical and denote hydrogen or a $C_1$-to $C_5$-alkyl group, in which case $R^4$ is a methyl group, or $R^2$ is hydrogen or $C_1$- to $C_5$-alkyl and $R^3$ and $R^4$, conjointly with the carbon atoms to which they are linked, represent a $C_5$- or $C_6$-cycloalkyl group or a group of the formula

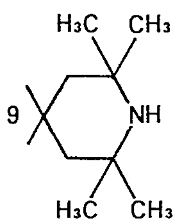

$R^5$ and $R^6$ are identical or different and represent hydrogen, $C_1$- to $C_{30}$-alkyl, a phenyl or naphthyl group which is unsubstituted or substituted by chlorine or $C_1$- to $C_4$-alkyl, or a $C_7$- to $C_{12}$-phenylalkyl group which is unsubstituted or substituted by $C_1$- to $C_4$-alkyl, or $R^5$ and $R^6$, conjointly with the carbon atom which is linked to them, denote a $C_5$- to $C_{18}$-cycloalkyl group which is unsubstituted or substituted by up to four $C_1$- to $C_4$-alkyl groups, or denote a group of the formula

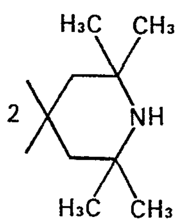

2. A process for the preparation of compounds as claimed in claim 1, which comprises reacting a polyalkyl-1-oxadiazaspirodecane of the formula (IV)

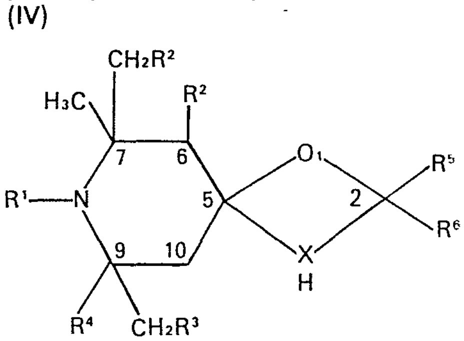 (IV)

in which $R^1$, $R^2$, $R^3$, $R^4$, $R^5$, $R^6$ and X have the meanings indicated in claim 1, with an epihalogenohydrin in a molar ratio of 1:1 to 1:5 in a two-phase system consisting of an organic solvent and an alkali metal hydroxide in the solid form or as an aqueous solution, in the presence of 0.1 to 5% by weight calculated on compound (IV) of a quaternary ammonium halide at 20 to 120°C, resulting products with n = 1 in the form of oxiranes, after which these oxiranes are polymerized at 70 to 240°C to give the ethers in which n = 2 to 50, if desired.

3. Use of the products as claimed in claim 1 for stabilizing synthetic polymers against the harmful effect of light, if appropriate together with previously known stabilizing substances in an amount of from 0.01 to 5 parts by weight, relative to the polymer.

4. Use as claimed in claim 3, wherein the polymer is a polyolefin, a halogen-containing polymer, a polyacrylate or polymethacrylate or a homopolymer of copolymer of polystyrene.

5. Synthetic polymers which have been stabilized against UV decomposition and which contain 0.01 to 5 parts by weight, relative to the polymer, of a stabilizer as claimed in claim 1.

**Claims** for the Contracting state: AT

1. Process for the preparation of ethers, containing polyalkylpiperidine groups, of the formula (I)

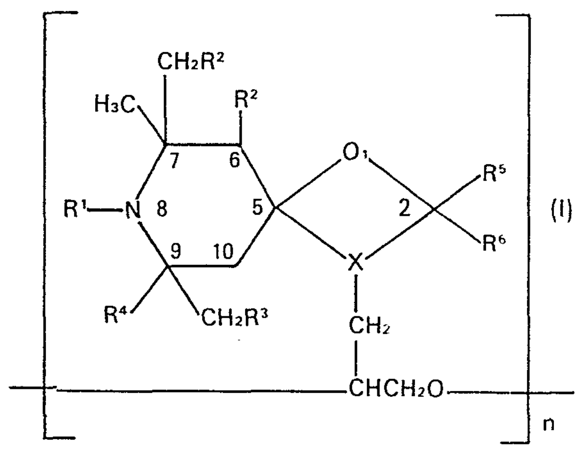 (I)

in which n is an integer from 1 to 50, when n equals 1, an oxirane ring being completed via the free bonds, X denotes a group of the formula (II) or (III)

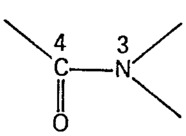 (II) 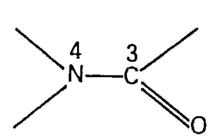 (III)

wherein the indices 3 and 4 indicate the ring positions in the diazaspirodecane system and one bond of the nitrogen is attached to the $CH_2$ group of the ether radial, $R^1$ is hydrogen, oxygen or $C_1$-

to $C_{12}$-alkyl, $R^2$ and $R^3$ are either identical and denote hydrogen or a $C_1$-to $C_5$-alkyl group, in which case $R^4$ is a methyl group, or $R^2$ is hydrogen or $C_1$- to $C_5$-alkyl and $R^3$ and $R^4$, conjointly with the carbon atoms to which they are linked, represent a $C_5$- or $C_6$-cycloalkyl group or a group of the formula

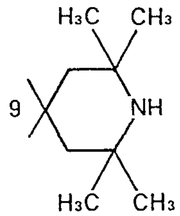

$R^5$ and $R^6$ are identical or different and represent hydrogen, $C_1$- to $C_{30}$-alkyl, a phenyl or naphthyl group which is unsubstituted or substituted by chlorine or $C_1$- to $C_4$-alkyl, or a $C_7$- to $C_{12}$-phenylalkyl group which is unsubstituted or substituted by $C_1$- to $C_4$-alkyl, or $R^5$ and $R^6$, conjointly with the carbon atom which is linked to them, denote a $C_5$- to $C_{18}$-cycloalkyl group which is unsubstituted or substituted by up to four $C_1$- to $C_4$-alkyl groups, or denote a group of the formula

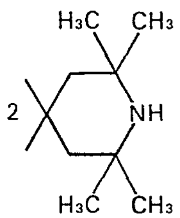

which comprises reacting a polyalkyl-1-oxadiazaspirodecane of the formula (IV)

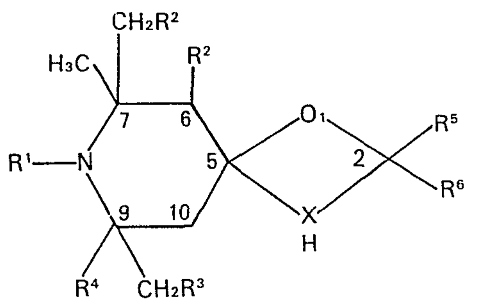 (IV)

in which $R^1$, $R^2$, $R^3$, $R^4$, $R^5$, $R^6$ and X have the meanings given above, with an epihalogenohydrin in a molar ratio of 1:1 to 1:5 in a two-phase system consisting of an organic solvent and an alkali metal hydroxide in the solid form or as an aqueous solution, in the presence of 0.1 to 5% by weight calculated on compound (IV) of a quaternary ammonium halide at 20 to 120°C, resulting products with n = 1 in the form of oxiranes, after which these oxiranes are polymerized at 10 to 240°C to give the ethers in which n = 2 to 50, if desired.

2. Use of the products as prepared according to claim 1 for stabilizing synthetic polymers against the harmful effect of light, if appropriate together with previously known stabilizing substances in an amount of from 0.01 to 5 parts by weight, relative to the polymer.

3. Use as claimed in claim 2, wherein the polymer is a polyolefin, a halogen-containing polymer, a polyacrylate or polymethacrylate or a homopolymer or copolymer of styrene.

4. Synthetic polymers which have been stabilized against UV decomposition and which contain 0.01 to 5 parts by weight, relative to the polymer, of a stabilizer according to claim 1.

**Revendications** pour les Etats contractants: BE, CH, DE, FR, GB, IT, LI, LU, NL, SE

1. Ethers contenant des radicaux polyalkylpipéridiniques qui répondent à la formule (I)

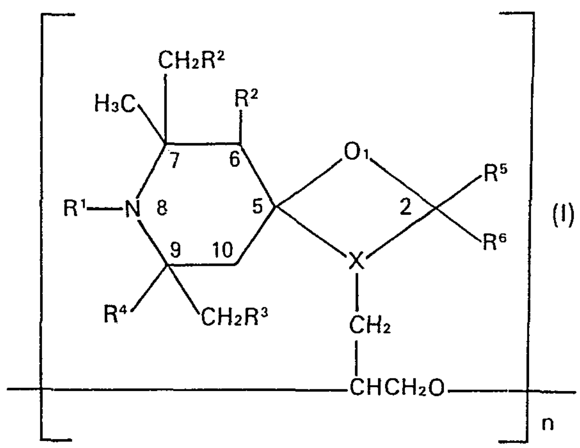 (I)

dans laquelle

n désigne un nombre entier de 1 à 50, les liaisons libres se réjoignant pour former un noyau d'oxiranne dans le cas où n est égal à 1,
X représente un radical répondant à l'une des formules II et III

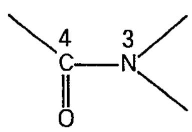 (II) 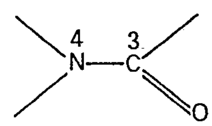 (III)

dans lesquelles les indices 3 et 4 désignent les positions dans le système cyclique du diazaspirodécane et une liaison de l'azote est reliée au groupe $-CH_2-$ du radical d'éther,

$R^1$ représente l'hydrogène, l'oxygène ou un alkyle en $C_1$–$C_{12}$

ou bien

$R^2$ et $R^3$ sont identiques et représentent chacun l'hydrogène ou un alkyle en $C_1$–$C_5$, auquel cas

$R^4$ représente un radical méthyle, ou encore

$R^2$ représente l'hydrogène ou un alkyle en $C_1$–$C_5$ et

$R^3$ et $R^4$ forment ensemble et avec les atomes de carbone auxquels ils sont liés un radical cycloalkyle en $C_5$ ou $C_6$ ou un radical de formule:

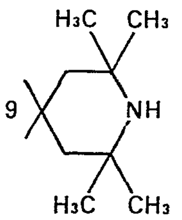

$R^5$ et $R^6$ sont identiques ou différents et représentent chacun l'hydrogène, un alkyle en $C_1$–$C_{30}$, un radical phényle ou naphtyle non substitué ou porteur d'un atome de chlore ou d'un alkyle en $C_1$–$C_4$, ou un phénylalkyle en $C_7$–$C_{12}$ non substitué ou porteur d'un alkyle en $C_1$–$C_4$, ou

$R^5$ et $R^6$ forment ensemble et avec l'atome de carbone qui les porte un radical cycloalkyle en $C_5$–$C_{18}$ non substitué ou porteur de 1 à 4 alkyles en $C_1$–$C_4$, ou un radical de formule:

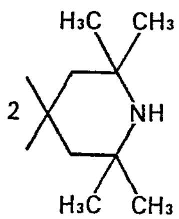

2. Procédé de préparation des composés selon la revendication 1, procédé caractérisé en ce qu'on fait réagir un polyalkyl oxa-1 diaza-spirodécane répondant à la formule IV

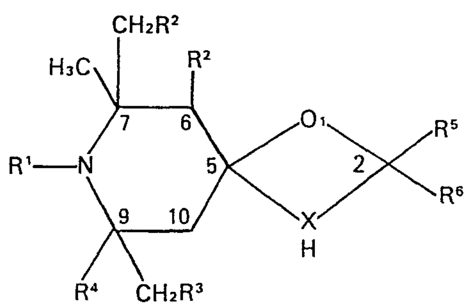

(IV)

dans laquelle $R^1$, $R^2$, $R^3$, $R^4$, $R^5$, $R^6$ et X ont les significations données à la revendication 1, avec une épihalogénhydrine, dans un rapport molaire compris entre 1:1 et 1:5, dans un système à deux phases constitué d'un solvant organique et d'un hydroxyde de métal alcalin à l'état solide ou en solution aqueuse, en présence de 0,1 à 5% en poids, par rapport au composé (IV), d'un halogénure d'ammonium quaternaire, à une température de 20 à 120°C, réaction qui donne des composés pour lesquels n est égal à 1 sous la forme de l'oxiranne, après quoi, si on le désire, on polymérise ces composés à une température de 100 à 240°C de manière à obtenir les éthers pour lesquels n est compris entre 2 et 50.

3. Application des composés selon la revendication 1 pour la stabilisation de polymères synthétiques contre l'action destructrice de la lumière, éventuellement associés à des composés stabilisants connus, en des quantités de 0,01 à 5 parties en poids par rapport au polymère.

4. Application selon la revendication 3, caractérisée en ce que le polymère est une polyoléfine, un polymère halogéné, un polyacrylate, un polyméthacrylate ou un homopolymère ou copolymère du styrène.

5. Polymères synthétiques stabilisés contre la destruction par les ultraviolets, polymères qui contiennent de 0,01 à 5 parties en poids, par rapport au polymère, d'un composé selon la revendication 1.

**Revendications** pour l'Etat contractant: AT

1. Procédé pour préparer des ethers contenant des radicaux polyalkyl-pipéridiniques qui répondent à la formule (I)

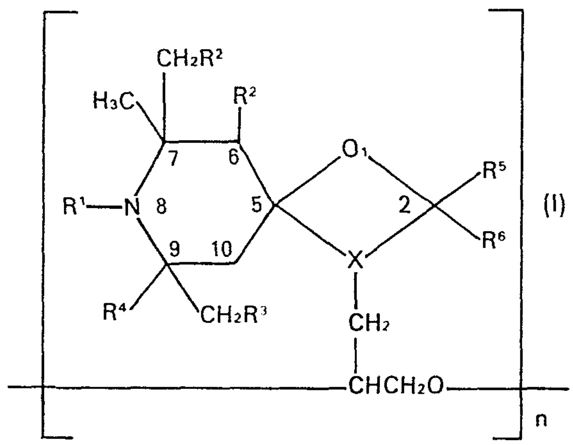

(I)

dans laquelle

n désigne un nombre entier de 1 à 50, les liaisons libres se réjoignant pour former un noyau d'oxiranne dans le cas où n est égal à 1,

X représente un radical répondant à l'une des formules II et III

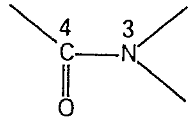

(II)

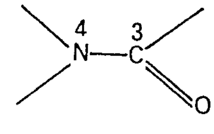

(III)

dans lesquelles les indices 3 et 4 désignent les positions dans le système cyclique du diazaspirodécane et une liaison de l'azote est reliée au groupe $-CH_2-$ du radical d'éther,

$R^1$ représente l'hydrogène, l'oxygène ou un alkyle en $C_1$–$C_{12}$

ou bien

$R^2$ et $R^3$ sont identiques et représentent chacun l'hydrogène ou un alkyle en $C_1$–$C_5$, auquel cas

$R^4$ représente un radical méthyle, ou encore

$R^2$ représente l'hydrogène ou un alkyle en $C_1$–$C_5$ et

$R^3$ et $R^4$ forment ensemble et avec les atomes de carbone auxquels ils sont liés un radical cycloalkyle en $C_5$ ou $C_6$ ou un radical de formule:

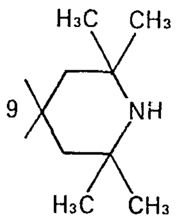

$R^5$ et $R^6$ sont identiques ou différents et représentent chacun l'hydrogène, un alkyle en $C_1$–$C_{30}$, un radical phényle ou naphtyle non substitué ou porteur d'un atome de chlore ou d'un alkyle en $C_1$–$C_4$, ou un phénylalkyle en $C_7$–$C_{12}$ non substitué ou porteur d'un alkyle en $C_1$–$C_4$, ou

$R^5$ et $R^6$ forment ensemble et avec l'atome de carbone qui les porte un radical cycloalkyle en $C_5$–$C_{18}$ non substitué ou porteur de 1 à 4 alkyles en $C_1$–$C_4$, ou un radical de formule:

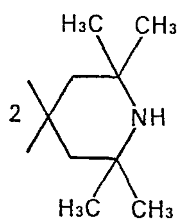

procédé caractérisé en ce qu'on fait réagir un polyalkyl oxa-1 diaza-spirodécane répondant à la formule IV

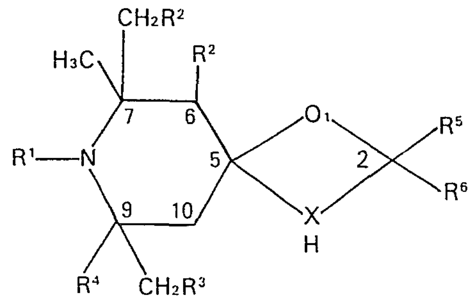

(IV)

dans laquelle $R^1$, $R^2$, $R^3$, $R^4$, $R^5$, $R^6$ et X ont les significations indiquées ci-dessus, à une température de 20 à 120°C, avec une épihalogénhydrine, dans un rapport molaire compris entre 1:1 et 1:5, dans un système à deux phases constitué d'un solvant organique et d'un hydroxyde de métal alcalin à l'état solide ou sous la forme d'une solution aqueuse, en présence de 0,1 à 5% en poids, par rapport au composé (IV), d'un halogénure d'ammonium quaternaire, réaction qui fournit des composés pour lesquels n est égal à 1 sous la forme de l'oxiranne, après quoi, si on le désire, on polymérise ces composés à une température de 100 à 240°C de manière à les convertir en les éthers pour lesquels n est un nombre de 2 et 50.

2. Application des composés préparés selon la revendication 1 pour la stabilisation de polymères synthétiques contre l'action destructrice de la lumière, éventuellement avec des composés stabilisants connus, en des quantités comprises entre 0,01 et 5 parties en poids par rapport au polymère.

3. Application selon la revendication 2, caractérisé en ce que le polymère est une polyoléfine, un polymère halogéné, un polyacrylate, un polyméthacrylate, ou un homopolymère ou copolymère du styrène.

4. Polymères synthétiques stabilisés contre la dégradation par les rayons ultraviolets, polymères qui contiennent de 0,01 à 5 parties en poids, par rapport au polymère, d'un composé préparé selon la revendication 1.